# FASCICULE DE BREVET EUROPEEN

(11) **EP 1 902 021 B1**
(45) Date de publication et mention de la délivrance du brevet: **25.12.2013**
(21) Numéro de dépôt: 06778629.3
(22) Date de dépôt: 21.06.2006
(51) Int. Cl.: C07D 201/04

(54) **PROCEDE DE SYNTHESE DU LAURYLLACTAME (L12) PAR REARRANGEMENT CATALYTIQUE EN PHASE GAZEUSE DE LA CYCLODODECANONE OXIME**
VERFAHREN ZUR SYNTHETISIERUNG VON LAURYL-LACTAM (L12) MITTELS KATALYTISCHER UMORDNUNG VON CYCLODODECANONOXIM IN EINER GASPHASE
METHOD FOR SYNTHESISING LAURYL LACTAM (L12) BY CATALYTICALLY REARRANGING CYCLODODECANONE OXIME IN A GASEOUS PHASE

(30) Priorité: 21.06.2005 FR 0506263; 23.09.2005 US 719842 P
(43) Date de publication de la demande: 26.03.2008
(73) Titulaire: ARKEMA FRANCE, 92700 Colombes (FR)
(72) Inventeur: LACROIX, Eric, F-69480 Amberieux d'Azergues (FR); HUB, Serge, F-69100 Villeurbanne (FR); HOLDERICH, Wolfgang, 67227 Frankenthal (DE); EICKELBERG, Wilm, 68935 Liederbach (DE); FAJULA, François, F-34820 Teyran (FR); DI RENZO, Francesco, F-34070 Montpellier (FR); BRANDHORST, Markus, F-69007 LYON (FR)
(74) Mandataire: Delprat, Corinne
(86) Numéro de dépôt international: PCT/FR2006/001399
(87) Numéro de publication internationale: WO 2006/136699

(56) Documents cités:
- EP-A- 1 193 251
- EP-A- 1 361 211
- WO-A-2004/037785
- JP-A- 2003 321 443
- JP-A- 2004 352 623
- US-A- 3 586 668

## Description

La présente invention se rapporte à un procédé de synthèse du lauryllactame (L12) par réarrangement catalytique de la cyclododécanone oxime. Ce réarrangement est réalisé en phase gazeuse en présence d'un catalyseur de type zéolithe.

Le caprolactame et le lauryllactame sont respectivement les précurseurs des polyamides 6 et 12. Au niveau industriel, le procédé classique pour synthétiser les lactames à partir de cétoximes cycliques est basé sur une réaction en milieu acide, par exemple l'acide sulfurique. Des quantités importantes d'acide sont nécessaires et la récupération du lactame impose de neutraliser le milieu par une base. Par conséquent, en plus des inconvénients liés à l'utilisation d'acide, celui-ci doit après réaction être neutralisé. Il ne peut donc pas être recyclé et conduit à une sous-production importante de rejets indésirables (sous-production de 3 à 5 tonnes de sulfate d'ammonium par tonne de lactame lorsque l'on utilise de l'acide sulfurique et que l'on neutralise le milieu à l'ammoniaque). Pour éviter cette sous-production de produits non valorisables, différents procédés ont été envisagés, notamment en utilisant la catalyse hétérogène.

Dans un article récent publié dans Studies in Surface Sciences and Catalysis (2001, 135, 3719-3726**),** Maria Climent et coll. décrivent la synthèse de zéolites délaminées et leurs applications en synthèse organique. Ils décrivent notamment l'utilisation de ces solides pour la transposition de Beckmann dans un procédé batch en phase liquide. Parmi les solides exemplifiés, la zéolithe β désaluminée est mentionnée, la conversion et la sélectivité obtenues avec cette zéolithe pour la transposition de la cyclododécanone oxime en L12 sont respectivement de 16 et 98 %. La sélectivité est donc élevée ; par contre, la conversion reste faible avec ce type de catalyseur. Par ailleurs, il n'y a aucune information sur la transposition de Beckmann en phase gazeuse.

La transposition de Beckmann a également été travaillée par catalyse hétérogène en phase gazeuse, notamment pour la synthèse du caprolactame. En effet, bien que ce soit des composés à point d'ébullition élevé (206-210°C pour la cyclohexanone oxime et 139°C/12 mmHg pour le caprolactame), des procédés ont été mis au point. En dehors de rechercher le bon catalyseur qui conduira à une activité catalytique élevée, la difficulté majeure est la stabilité du catalyseur qui se désactive rapidement suite à une adsorption/décomposition des composés organiques. Dans le cas de la transposition de la cyclododécanone oxime, les composés organiques sont encore plus lourds que ceux de la série à six atomes de carbone. C'est pourquoi, même si quelques essais de courtes durées sont décrits dans la littérature, un procédé industriel paraissait difficilement envisageable compte tenu de la difficulté à maintenir l'activité catalytique.

Le brevet FR 1562298 (équivalent à US 3586668**)** décrit la synthèse de lactame ,par réarrangement en phase gazeuse sur des systèmes catalytiques consistant en un mélange de trioxyde de bore ou d'acide borique et de carbone hautement dispersé (dimension des particules inférieure à 0,1 mm) et en présence d'eau. Ce brevet décrit et exemplifie surtout la synthèse du caprolactame ; cependant le dernier exemple (exemple 8) décrit la synthèse du lauryllactame sur un catalyseur dont la taille de particule est comprise entre 1 et 2 mm. L'essai décrit dans cet exemple est de courte durée (5 heures 15 minutes) et, bien que la teneur en L12 dans le mélange final soit mentionnée (86 %), il n'est pas possible d'en déduire le rendement de la réaction puisque la masse de produit obtenue n'est pas indiquée. Par ailleurs, aucune information sur la stabilité et la durée de vie du catalyseur n'est mentionnée.

Dans la demande de brevet JP 48012754 A (dépôt No 44-76676 du 7 octobre 1969 au nom d'Asahi Chemical Industry) on mentionne les problèmes rencontrés lors d'une transposition de Beckmann en phase gazeuse sur un catalyseur hétérogène. Entre autres, les inventeurs soulèvent le problème de désactivation très rapide du catalyseur lié à la décomposition thermique et la polymérisation des composés organiques sur le solide. Pour remédier à ces difficultés, notamment pour la cyclododécanone oxime, les inventeurs préconisent l'utilisation de catalyseurs constitués d'acide borique déposé sur un support poreux tel que les diatomées. L'exemple 1 de ce brevet décrit le réarrangement de la cyclododécanone oxime sur un catalyseur de ce type. Bien que répété 30 fois, le test est de courte durée (5 minutes). Il est mentionné que la conversion est stable si la température est de 300°C ; par contre aucune information n'est indiquée sur la sélectivité ou le rendement en L12.

Dans les brevets US 6051706 et US 6071844 on revendique la synthèse du caprolactame par catalyse hétérogène en phase gazeuse sur des zéolithes β ou de type MFI. Dans le souci d'améliorer les performances des catalyseurs notamment de réduire leur désactivation, d'une part la cyclohexanone oxime est dissoute dans de l'éthanol et d'autre part, les catalyseurs sont modifiés (utilisation de zéolithes contenant du bore et traitement des MFI pour éliminer tout ou partie des atomes métalliques centraux). Malgré cela, les inventeurs ont été amenés à travailler sur la régénération du catalyseur en présence d'air ou d'azote. La synthèse du L12 n'est pas mentionnée dans ces brevets puisqu'ils sont limités à la synthèse du caprolactame.

La demande de brevet WO 2004 037795 décrit le réarrangement catalytique de la cyclododécanone oxime en lauryllactame (L12) soit (i) en phase gazeuse soit (ii) en phase liquide en présence d'un catalyseur qui est un silicate monolaminaire à caractère acide. Ce catalyseur est préparé par délamination du précurseur d'une zéolithe à structure laminaire, ce n'est donc pas une zéolithe.

Ainsi l'art antérieur a décrit le réarrangement catalytique de la cyclododécanone oxime en lauryllactame (L12) soit (i) en phase gazeuse sur des catalyseurs qui ne sont pas des zéolithes (FR 1562298 et JP 48012754 A) soit (ii) en phase liquide sur des zéolithes (Studies in Surface Sciences and Catalysis (2001, 135, 3719-3726)). Une zéolithe est définie comme un matériau microporeux ayant une structure principale inorganique à 3 dimensions composée de tétraèdres liés par une arête commune (fully linked corner-sharing tetraedra) voir en particulier "Nomenclature of structural and compositional characteristic of ordered microporous and mesoporous materials with inorganic hosts. L.B. Mc Cusker, F. Liebau, G. Engelhardt, Pure Appl. Chem., 73,, pp 381-394, 2001"

On a maintenant trouvé qu'on pouvait effectuer le réarrangement catalytique de la cyclododécanone oxime en lauryllactame en phase gazeuse sur une zéolithe.

### [Brève description de l'invention]

La présente invention concerne un procédé de préparation du lauryllactame dans lequel on effectue un réarrangement de Beckmann de la cyclododécanone oxime en phase gazeuse à une température comprise entre 180 et 450°C à une pression comprise entre 50 et 70 mbars absolus, en présence d'une zéolithe.

Avantageusement la zéolithe est une zéolithe présentant une distribution poreuse dans la zone des larges pores (par exemple pores dont l'ouverture est délimitée par des cycles à 12 tétraèdres).

Selon une forme avantageuse de l'invention le procédé comprend les étapes suivantes :
a) la cyclododécanone oxime est soit mise en solution dans un solvant choisi parmi les alcools et les hydrocarbures soit à l'état fondu,
b) le courant de l'étape a) est vaporisé et mis en contact avec la zéolithe éventuellement à l'aide d'un gaz porteur,
c) on sépare le lauryllactame du solvant, du gaz porteur éventuel et de la cyclododécanone oxime éventuelle qui n'a pas été réarrangée (convertie).

Selon une autre forme la zéolithe possède l'aluminium et/ou le bore comme hétéroatome de réseau.

Selon une autre forme la zéolithe est une zéolithe ayant initialement l'aluminium et/ou le bore comme hétéroatome de réseau et ayant subi un traitement de désalumination /déboration.

Selon une autre forme la zéolithe est une zéolithe β.

Selon une autre forme la zéolithe est une zéolithe β ayant l'aluminium et/ou le bore comme hétéroatome de réseau.

Selon une autre forme la zéolithe est une zéolithe β ayant initialement l'aluminium et/ou le bore comme hétéroatome de réseau et ayant subi un traitement de désalumination /déboration.

Dans la suite du texte la zéolithe est parfois désignée par le terme "catalyseur".

Ces conditions opératoires permettent de réduire l'accumulation de L12 (ou de ces dérivés) sur le catalyseur, tout en minimisant la formation de coke qui désactiverait le catalyseur. De manière à travailler à l'état gazeux sans atteindre des températures trop élevées qui conduiraient inévitablement à une décomposition des organiques et à une désactivation irréversible du catalyseur, la réaction est opérée sous pression réduite. Il est recommandé d'effectuer périodiquement une désorption du L12 et de ses dérivés adsorbés à la surface du catalyseur. Cette désorption est assurée par des traitements à l'air ou sous gaz inerte à une température supérieure à la température de la zéolithe pendant la transposition de Beckmann. Ces traitements permettent d'éviter la transformation des produits adsorbés en « coke »; qui entraînerait une désactivation irréversible du catalyseur.

### [Description détaillée de l'invention]

**S'agissant de la température à laquelle se fait la transposition,** un fonctionnement à des températures inférieures à 180°C conduit à une désactivation rapide et irréversible du catalyseur. A l'opposé, des températures supérieures à 450°C conduisent à une dégradation des organiques avec là aussi une désactivation irréversible du catalyseur. Dans la fourchette de températures comprises entre 180 et 450°C, il est possible de synthétiser du lauryllactame avec une sélectivité généralement supérieure à 70%. De manière à obtenir également une bonne conversion tout en protégeant le catalyseur d'une désactivation irréversible suite à des températures trop élevées, nous préconisons de travailler dans la fourchette 225-400°C. L'intervalle 225-375°C étant particulièrement préféré.

**S'agissant de la pression** de fonctionnement, compte tenu que la cyclododecanone oxime et le lauryllactame sont difficiles à vaporiser et risquent de former des oligomères, voire du coke sur le catalyseur, il est préférable de travailler sous pression réduite. Là aussi, il faut trouver le meilleur compromis, puisqu'une faible pression permet de désorber plus facilement les composés organiques donc d'améliorer la sélectivité. Par contre, elle limite l'adsorption du substrat sur le catalyseur et par conséquent réduit la conversion. Compte tenu de la fourchette de températures sélectionnée, on travaille dans le domaine 50- 700 mbars absolus.

**S'agissant de la zéolithe** ces produits sont connus en eux mêmes et sont disponibles dans le commerce. A titre d'exemple on peut utiliser une zeolithe USY, une zéolithe qui possède l'aluminium et/ou le bore comme hétéroatome de réseau, une zéolithe ayant initialement l'aluminium et/ou le bore comme hétéroatome de réseau et ayant subi un traitement de désalumination /déboration, une zéolithe β, une zéolithe β ayant l'aluminium et/ou le bore comme hétéroatome de réseau, une zéolithe β ayant initialement l'aluminium et/ou le bore comme hétéroatome de réseau et ayant subi un traitement de désalumination /déboration. Ce traitement de désalumination/déboration appliqué aux zéolithes ou aux zéolithes β permet d'améliorer sensiblement les performances du catalyseur notamment du point de vue sélectivité. Pour procéder à la désalumination, voire la déboration de ces zéolithes, il existe de nombreuses méthodes décrites dans la littérature ; il s'agit de traitements hydrothermiques ou chimiques de la zéolithe. A titre non limitatif, on peut citer la méthode décrite dans le brevet EP 488867. L'avantage du traitement acide décrit dans ce brevet est qu'il permet de conserver la cristallinité de la zéolithe. Il est particulièrement bien approprié pour la désalumination/déboration de la zéolithe β. Les conditions opératoires retenues pour ce traitement permettent d'aller plus ou moins loin dans la désalumination/déboration de la zéolithe et son efficacité est mesurée par les ratios atomiques Si/Al ou Si/B des solides obtenus. La zéolithe utilisée peut initialement contenir du bore. Le traitement pour éliminer une partie des atomes d'aluminium de la zéolithe conduit également à une élimination partielle du bore. La présence de bore résiduel n'affecte pas les performances du catalyseur en terme de sélectivité et contribue même à une amélioration de la conversion. Une zéolithe ne contenant initialement que du bore comme hétéroatome de réseau, conduit après un traitement de déboration à une conversion plus faible ; cependant la sélectivité reste toujours très bonne (supérieure à 90 % dans certaines conditions opératoires).

S'agissant des zéolithes (éventuellement beta) ayant initialement l'atome d'aluminium comme hétéroatome de réseau on peut effectuer une désalumination conduisant à un rapport atomique Si/Al supérieur à 50, avantageusement supérieur à 80 et de préférence supérieur à 150.

S'agissant des zéolithes (éventuellement beta) ayant initialement l'atome de bore comme hétéroatome de réseau on peut effectuer une déboration conduisant à un rapport atomique Si/B supérieur à 20 et avantageusement supérieur à 40.

S'agissant des zéolithes (éventuellement beta) ayant initialement l'atome d'aluminium et l'atome de bore comme hétéroatome de réseau on peut effectuer une désalumination/déboration conduisant à un rapport Si/Al supérieur à 50 et un rapport Si/B supérieur à 20, avantageusement à un rapport Si/Al supérieur à 150 et un rapport Si/B supérieur à 30.

**S'agissant du solvant,** La cyclododécanone oxime peut être mise en solution dans un solvant choisi parmi les alcools et les hydrocarbures. Il est recommandé de choisir le solvant pour permettre de solubiliser les composés organiques et avoir une stabilité du solvant acceptable dans les conditions opératoires retenues pour la réaction (température, zéolithe ...). De manière générale, les alcools qui peuvent se décomposer en présence de zéolithes, sont nettement plus stables en présence de zéolithes désaluminées et/ou déboronées. Parmi les alcools pouvant être utilisés comme solvant de la cyclododécanone oxime, on citera à titre non limitatif le méthanol, l'éthanol, l'isopropanol. De même, des hydrocarbures peuvent être utilisés seuls ou en mélanges, ce qui permet d'avoir une forte dépendance de la solubilité des produits avec la température. Cette dernière propriété étant importante pour la récupération et la purification des produits finaux. L'utilisation d'isopropanol ou de mélanges isopropanol/cyclohexane, éthanol/cyclohexane comme solvant est particulièrement préférée. Par ailleurs, afin d'augmenter la solubilité de la cyclododécanone oxime dans le solvant et donc d'augmenter la productivité de la transposition, il est possible de préchauffer le mélange cyclododécanoneoxime/solvant avant son introduction dans la partie réactionnelle.

**S'agissant du gaz porteur,** on peut citer à titre d'exemple l'azote, l'argon et l'helium.

**Quant à l'étape c),** la séparation peut s'effectuer par tout moyen.

**Quant à la "régénération" du catalyseur,** on désigne ainsi les traitements qui permettent la désorption du L12 et de ses dérivés adsorbés à la surface du catalyseur (de la zéolithe). Les catalyseurs (zéolithes) et les conditions opératoires décrites ci dessus et dans les exemples conduisent à de bonnes performances catalytiques (sélectivité et conversion) et permettent de limiter l'accumulation de composés organiques sur le catalyseur. Cependant, il est impossible de supprimer totalement cette accumulation de produits organiques sur le catalyseur. C'est pourquoi, afin d'améliorer la durée de vie du catalyseur et d'éviter des désactivations irréversibles, il est préconisé de procéder à une régénération du catalyseur dès que l'on observe une baisse significative (10 à 20 %) du rendement. Cette régénération est assurée par un balayage ou/et mise sous vide du catalyseur en absence des réactifs organiques. Une température supérieure à la température à laquelle on a fait la réaction de transposition est préconisée. C'est pourquoi, la régénération est conduite dans une fourchette de températures comprises entre 350 et 650°C et plus particulièrement entre 400 et 600°C. La fourchette de températures 450-590°C étant particulièrement préférée.

Cette régénération peut être réalisée sous pression atmosphérique ou sous pression réduite. De même, elle peut être réalisée sous balayage de gaz inertes tels que l'azote ou sous oxygène ou sous un mélange des deux tels que l'air. La durée de la régénération peut être déterminée en suivant la perte de masse du catalyseur due à la désorption des composés organiques adsorbés sur le catalyseur. Elle nécessite en général plusieurs heures si l'on prend en compte les phases de montée et de baisse de la température. La regeneration peut aussi être effectuée sous vide.

**S'agissant du dispositif** dans lequel on effectue la présente transposition ainsi que la préparation des réactifs et la récupération du lauryllactame, on utilise les appareillages usuels. Plus particulièrement la réaction de transposition sur la zéolithe peut être faite sur des réacteurs dits "en lit fixe", en "lit fluide" ou en "lit circulant". Compte tenu de la nécessité de régénérer le catalyseur régulièrement, il peut être avantageux d'utiliser plusieurs ensembles réactionnels avec une partie d'entre eux en production alors que les autres sont en phase de régénération, puis inversement.

### [Exemples]

### Synthèse de zéolithes β

### Synthèse d'une zéolithe β avec l'aluminium comme hétéroatome de réseau : CAT 1

1,1 g de soude (Carlo Erba) sont dissous dans 78,6 g d'eau, puis on ajoute successivement sous agitation 45 g d'une solution à 35% d'hydroxyde de tétraéthylammonium (Aldrich), 0.48 g de NaAlO2 (Carlo Erba). Après dissolution, on ajoute toujours sous agitation 18 g de silice Zéosil 175 MP. Après une étape de mûrissement sous agitation de 4 heures à température ambiante, le mélange est porté, pendant 48 heures en statique dans un autoclave, à une température de 150°C. Le mélange obtenu est filtré puis lavé à l'eau jusqu'à l'obtention d'un pH 9,4. Le solide obtenu est séché à 100°C pendant 12 heures.

L'analyse élémentaire du solide sec indique un ratio atomique Si/Al de 11

### Synthèse d'une zéolithe β avec l'aluminium et le bore comme hétéroatomes de réseau : CAT 2

0,75 g de NaOH (Carlo Erba) sont dissous dans 24 g d'eau, puis on ajoute successivement sous agitation 0.059 g de NaAlO2 (Carlo Erba), 0,492 g de Na2B4O7 (Carlo Erba). Après dissolution, on ajoute sous agitation 45 g d'une solution à 35% d'hydroxyde de tétraéthylammonium (Aldrich), puis 18 g de silice Zéosil 175 MP. Après une étape de mûrissement sous agitation de 4 heures à température ambiante, le mélange est porté, pendant 48 heures en statique dans un autoclave, à une température de 150°C. Le mélange obtenu est filtré puis lavé à l'eau jusqu'à l'obtention d'un pH de 9,1, puis centrifugé. Le gâteau est finalement séché à 100°C pendant 14 heures.

L'analyse élémentaire du solide obtenu indique un ratio atomique Si/Al de 41 et Si/B de 19,8

### Synthèse d'une zéolithe β avec le bore comme hétéroatome de réseau : CAT 3

3, 48 g d'hydroxyde de bore (B(OH)3 - Aldrich), 1.43 g de soude, 26,6 g de silice FK700 (Degussa) et 27,2 g d'une solution aqueuse à 40% d'hydroxyde de tétraéthylammonium (Fluka) sont ajoutés à 183,6 ml d'eau et maintenu sous agitation à température ambiante pendant une nuit (13H). Puis, 31,9 g de bromure de tétraéthylammonium sont ajoutés et le mélange est maintenu sous agitation pendant 5 h. Le mélange est porté pendant 240H à 150°C sous pression autogène dans un autoclave muni d'une enveloppe Teflon^{®}. Après filtration, les cristaux obtenus sont calcinés une première fois à 400°c sous flux d'ammoniac (3l/h). Après retour à température ambiante, le solide est lavé trois fois pendant 24 h avec une solution de chlorure d'ammonium 1M. Après filtration, le solide obtenu est calciné à 400°C sous azote.

L'analyse du solide ainsi synthétisé révèle un ratio atomique Si/B de 16

### Désalumination

### Désalumination de CAT 1

Pour extraire une partie de l'aluminium, le CAT 1, dont la synthèse est décrite précédemment, est traité à 130°C (reflux) en présence d'acide nitrique 70 %. Un traitement à reflux pendant 5 heures, suivi d'un lavage à l'acide nitrique 17% puis à l'eau conduit après séchage sous air à 80°C à un solide ayant un rapport atomique Si/Al de 150 (Catalyseur CAT1 désalumination AI 1 (abrégée en désAl 1)). Le solide obtenu est ensuite calciné sous air à 550°C pendant 8 heures (vitesse de montée en température : 2°C/minute).

Un traitement identique mais avec une étape de reflux pendant 6,5 heures conduit après lavage, séchage et calcination à un solide ayant un ratio atomique Si/Al = 180 (catalyseur CAT1 désalumination Al2 (abrégée en dés Al2)).

### Désalumination-déboration de CAT 2

Pour éliminer une partie des hétéroatomes de réseau du catalyseur CAT 2, ce catalyseur est traité à reflux (130 °C) d'acide nitrique 70 % pendant 5 heures. Après lavage à l'acide nitrique 17%, puis à l'eau et séchage, le solide est calciné sous air à 550°C pendant 8 heures (vitesse de montée en température : 2°C/minute). On obtient ainsi un solide, dont l'analyse élémentaire indique un rapport atomique Si/Al de 170 et Si/B de 37 (Catalyseur CAT2 désaluminé-déboré (abrégé en dés AlB 1)).

### Déboration de CAT 3

Le catalyseur CAT 3 est soumis à un traitement d'une heure à température ambiante avec une solution HCl (pH 6). Après lavage à l'eau, puis séchage l'analyse élémentaire du solide révèle un ratio atomique Si/B de 32 et Si/Al > 1500 (CAT 3 dés B1)

### Essais de transposition :

Dans un appareillage constitué d'un ensemble réactionnel pouvant fonctionner à pression atmosphérique ou sous pression comprenant une chambre de vaporisation et un réacteur, on alimente par le biais d'une pompe, de la cyclododécanone oxime en solution dans un solvant. La pression réduite dans la partie réactionnelle est assurée par une pompe à vide équipée d'un manomètre. Sauf indication contraire, la charge de catalyseur utilisée dans le réacteur lit fixe est de un gramme. L'ensemble des produits de réaction est récupéré dans un piège à azote liquide.

### Test 1 : Test du catalyseur CAT 1 (catalyseur non désaluminé)

Pour cet essai la cyclododécanone oxime est solubilisée dans de l'isopropanol à température ambiante (3g d'oxime/100 g d'isopropanol). La température dans le lit catalytique est fixée à 325°C et la pression de fonctionnement est établie à 50 mbars. Dans ces conditions opératoires le mélange cyclododécanoneoxime/isopropanol est injecté accompagné d'un gaz vecteur (3,5 NI/H d'azote), ce qui conduit à une vitesse spatiale de 0,3 g d'oxime/g de catalyseur.H. Après mise en régime de l'installation (une heure), le brut réactionnel est piégé pendant une heure. L'analyse de ce mélange conduit au résultat suivant : conversion de l'oxime 85 % et sélectivité en lauryllactame 68%.

### Test 2 : Test du catalyseur CAT 1 dés Al1

Ce test 2 est réalisé dans des conditions opératoires identiques à celles du test 1 à l'exception du catalyseur CAT 1 qui est remplacé par le catalyseur CAT1 dés Al1. L'analyse du brut réactionnel collecté pendant la deuxième heure de test conduit au résultat suivant : conversion de la cyclododécanone oxime : 40 % sélectivité en lauryllactame : 99 %.

### Test 3 : Test du catalyseur CAT 1 dés Al2

Ce test 3 est réalisé dans des conditions opératoires identiques à celles du test 1 à l'exception du catalyseur CAT 1 qui est remplacé par le catalyseur CAT1 dés Al2. L'analyse du brut réactionnel collecté pendant la deuxième heure de test conduit au résultat suivant : conversion de la cyclododécanone oxime : 48 % sélectivité en lauryllactame : 99 %.

### Test 4 : Test du catalyseur CAT 2 dés AlB1

Ce test 4 est réalisé dans des conditions opératoires identiques à celles du test 1 à l'exception du catalyseur CAT 1 qui est remplacé par le catalyseur CAT2 dés AlB1. L'analyse du brut réactionnel collecté pendant la deuxième heure de test conduit au résultat suivant : conversion de la cyclododécanone oxime : 89 % sélectivité en lauryllactame : 99,5 %.

### Test 5 : Test du catalyseur CAT 3 dés B1

Ce test 5 est réalisé dans des conditions opératoires identiques à celles du test 1 à l'exception du catalyseur CAT 1 qui est remplacé par le catalyseur CAT3 dés B1 L'analyse du brut réactionnel collecté pendant la deuxième heure de test conduit au résultat suivant : conversion de la cyclododécanone oxime : 28 % sélectivité en lauryllactame : 92 %

Les zéolithes β utilisées dans ces exemples ont initialement l'aluminium ou le bore comme hétéroatome de réseau. Les meilleurs résultats sont obtenus avec une zéolite contenant initialement de l'aluminium et du bore et ayant été soumise à un traitement de désalumination/déboration.

### Test 6 : Durée de vie

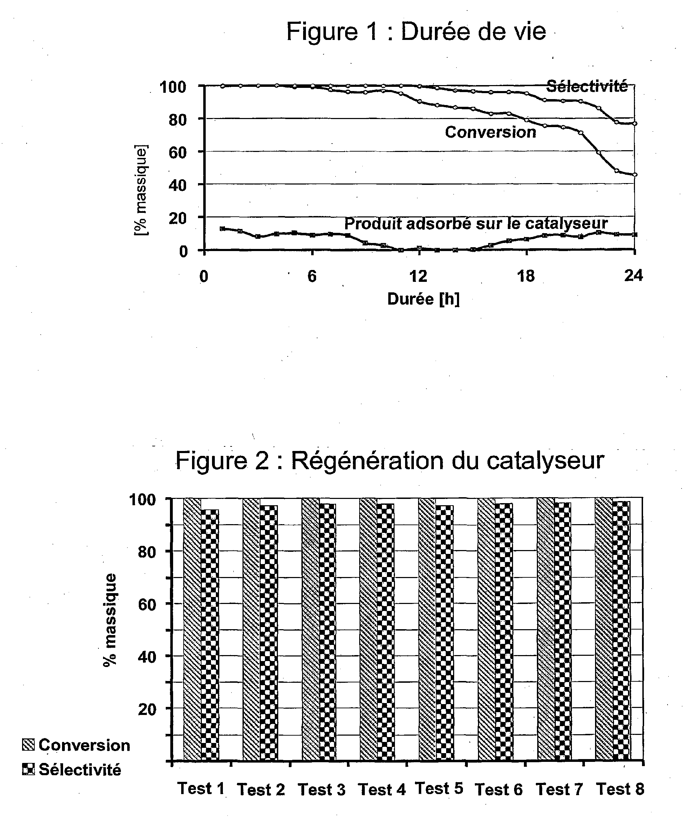

Pour cet essai la cyclododécanone oxime est solubilisée dans de l'isopropanol à température ambiante (3g d'oxime/100 g d'isopropanol). Ce mélange oxime-isopropanol est injecté dans la chambre de vaporisation à un débit de 10 g/h. Le catalyseur utilisé (3g) est le CAT1 dés Al2 et la température dans le lit catalytique est fixée à 325°C. Par ailleurs, la pression de fonctionnement est établie à 50 mbars. Après mise en régime de l'installation (une heure), le brut réactionnel est piégé, pesé et analysé toutes les heures. La fig 1 suivante illustre l'évolution de la conversion, de la sélectivité en L12 dans le brut réactionnel piégé en sortie de réacteur et le bilan matière permet de quantifier la masse de produit adsorbé sur le catalyseur.

Un bilan sur les douze premières heures de test montre que sur les 3.6 g d'oxime introduit dans la chambre de vaporisation, on récupère 3,36 g de produits dans le piège dont l'analyse montre que ce mélange est très majoritairement constitué de L12 (>99%). La présence de traces de cyclododécanone est également observée. Le produit manquant est resté adsorbé sur le catalyseur. Des essais complémentaires avec régénération montrent qu'il est possible lorsque la régénération est conduite avant une baisse significative de la sélectivité (régénération avant que la sélectivité soit en dessous de 85%) de récupérer 96 % du produit adsorbé sur le catalyseur et que ce produit est très majoritairement du L12 (>95%). Par conséquent sur cette période de 12 heures, un bilan complet incluant une «régénération préventive» à l'azote avec piégeage des produits désorbés conduit à un rendement en L12 de l'ordre de 96 %. Dans de telles conditions opératoires, la productivité en L12 est de 96 g/H.kg de catalyseur.

Les douze premières heures de test sont suivies d'une phase de 4 à 5 heures durant laquelle la sélectivité baisse légèrement, accompagnée d'une baisse progressive de la conversion jusqu'à environ 80%. Durant cette phase intermédiaire, il n'y a pas d'accumulation de produit sur le catalyseur. La troisième phase (après 16 heures de test) s'illustre par une désactivation plus rapide du catalyseur, notamment de la conversion. De nouveau il y a accumulation de composés organiques sur le catalyseur et les essais de régénération (dans les conditions décrites ci-après) entrepris sur un catalyseur à ce stade montrent qu'il n'est pas possible de désorber la totalité des produits accumulés et l'on observe une désactivation irréversible du catalyseur.

### Test 7 : Régénération du catalyseur

Une même charge de catalyseur (3 g) CAT1 dés Al2 a été testée successivement en réaction (P : 50 mbars, T = 300°C, oxime introduite en solution dans l'isopropanol, durée 2 heures avec piégeage du brut réactionnel durant la deuxième heure) et en régénération (balayage sous air à 550°C, durée totale de la régénération 12 heures (incluant la montée de la température à 550°C et la baisse à 300°C, pression atmosphérique). La fig 2 suivante traduit l'évolution de la conversion et de la sélectivité (analyse basée sur le brut réactionnel piégé) en fonction des phases successives de tests en réaction.

Après 8 tests en réaction, on observe que pour tous ces tests la conversion de la cyclododécanone oxime est totale. La sélectivité en L12 initialement de 95 % progresse légèrement pour atteindre 98 % lors du huitième test en réaction. En raison du temps nécessaire à la montée de la température pour atteindre le pallier de 550°C, puis la baisse de celle-là pour revenir à la température de réaction, il n'a pas été possible d'écourter la phase de régénération. A l'inverse une telle procédure de régénération (durée totale 12 heures) appliquée à un catalyseur ayant travaillé en réaction pendant 12 heures permet de maintenir l'activité catalytique pour le cycle suivant de réaction.

Le test a été interrompu après 8 cycles test/régénération sans observer de signes significatifs de désactivation du catalyseur.

Sur la base de ces résultats, il peut donc être envisagé un procédé avec plusieurs réacteurs en parallèle dont certains sont en phase de réaction alors que d'autres sont en phase de régénération.

### Test 8 : avec une zéolithe USY (autre famille de zéolithes que les zéolithes beta).

La zeolithe USY est commercialisée par Grace elle a un ratio atomique Si/Al de 35. Conditions de test identiques au test 1 plus haut mais en utilisant de la zéolithe USY à la place de la zéolithe beta.

Après mise en régime de l'installation (1 heure), le brut réactionnel est piégé pendant 1 heure. L'analyse de ce mélange révèle une conversion de l'oxime de 74% et une sélectivité en L12 de 75%.

## Revendications

1. Procédé de préparation du lauryllactame dans lequel on effectue un réarrangement de Beckmann de la cyclododécanone oxime en phase gazeuse à une température comprise entre 180 et 450°C, à une pression comprise entre 50 et 700 mbars absolus, en présence d'un matériau microporeux ayant une structure principale inorganique à 3 dimensions composée de tétraèdres liés par une arête commune, appelé zéolithe.

2. Procédé selon la revendication 1 dans lequel la zéolithe est une zéolithe présentant une distribution poreuse dans la zone des larges pores.

3. Procédé selon la revendication 1 ou 2 dans lequel :
a) la cyclododécanone oxime est soit mise en solution dans un solvant choisi parmi les alcools et les hydrocarbures soit à l'état fondu,
b) le courant de l'étape a) est vaporisé et mis en contact avec la zéolithe éventuellement à l'aide d'un gaz porteur,
c) on sépare le lauryllactame du solvant, du gaz porteur éventuel et de la cyclododécanone oxime éventuelle qui n'a pas été convertie.

4. Procédé selon l'une quelconque des revendications 1 à 3 dans lequel la température du réarrangement est comprise entre 225 et 400°C.

5. Procédé selon la revendication 4 dans lequel la température est comprise entre 225 et 375°C.

6. Procédé selon l'une quelconque des revendications précédentes dans lequel la zéolithe est une zéolithe USY.

7. Procédé selon l'une quelconque des revendications 1 à 5 dans lequel la zéolithe est choisie parmi une zéolithe qui possède l'aluminium et/ou le bore comme hétéroatome de réseau, une zéolithe ayant initialement l'aluminium et/ou le bore comme hétéroatome de réseau et ayant subi un traitement de désalumination /déboration, une zéolithe β, une zéolithe β ayant l'aluminium et/ou le bore comme hétéroatome de réseau, une zéolithe β ayant initialement l'aluminium et/ou le bore comme hétéroatome de réseau et ayant subi un traitement de désalumination /déboration.

8. Procédé selon la revendication 7 dans lequel la zéolithe est une zéolithe ayant initialement l'aluminium et/ou le bore comme hétéroatome de réseau et ayant subi un traitement de désalumination /déboration.

9. Procédé selon la revendication 7 dans lequel la zéolithe est une une zéolithe β ayant initialement l'aluminium et/ou le bore comme hétéroatome de réseau et ayant subi un traitement de désalumination/déboration.

10. Procédé selon la revendication 8 ou 9 dans lequel la zéolithe éventuellement beta ayant initialement l'aluminium comme hétéroatome de réseau subit une désalumination conduisant à un rapport atomique Si/Al supérieur à 50.

11. Procédé selon la revendication 10 dans lequel la zéolithe éventuellement beta ayant initialement l'atome d'aluminium comme hétéroatome de réseau subit une désalumination conduisant à un rapport atomique Si/Al supérieur à 80.

12. Procédé selon la revendication 11 dans lequel la zéolithe éventuellement beta ayant initialement l'atome d'aluminium comme hétéroatome de réseau subit une désalumination conduisant à un rapport atomique Si/Al supérieur à 150.

13. Procédé selon la revendication 8 ou 9 dans lequel la zéolithe éventuellement beta ayant initialement l'atome de bore comme hétéroatome de réseau subit une déboration conduisant à un rapport atomique Si/B supérieur à 20.

14. Procédé selon la revendication 13 dans lequel la zéolithe éventuellement beta ayant initialement l'atome de bore comme hétéroatome de réseau subit une déboration conduisant à un rapport atomique Si/B supérieur à 40.

15. Procédé selon la revendication 8 ou 9 dans lequel la zéolithe éventuellement beta ayant initialement l'atome d'aluminium et l'atome de bore comme hétéroatome de réseau subit une désalumination/déboration conduisant à un rapport Si/Al supérieur à 50 et un rapport Si/B supérieur à 20.

16. Procédé selon la revendication 15 dans lequel la zéolithe éventuellement beta ayant initialement l'atome d'aluminium et l'atome de bore comme hétéroatome de réseau subit une désalumination/déboration conduisant à un rapport Si/Al supérieur à 150 et un rapport Si/B supérieur à 30.

17. Procédé selon l'une quelconque des revendications 3 à 16 dans lequel la cyclododécanone oxime est introduite à l'état fondu.

18. Procédé selon l'une quelconque des revendications 3 à 16 dans lequel la cyclododécanone oxime est mise en solution dans un solvant choisi parmi les alcools et les hydrocarbures et l'alcool est choisi parmi le méthanol, l'éthanol, l'isopropanol.

19. Procédé selon la revendication 18 dans lequel le solvant est choisi parmi les mélanges isopropanol/cyclohexane et éthanol/cyclohexane.

20. Procédé selon l'une quelconque des revendications précédentes dans lequel on effectue une régénération de la zéolithe.

21. Procédé selon la revendication 20 dans lequel on effectue une régénération de la zéolithe dès que l'on observe une baisse du rendement comprise entre 10 et 20%.

## Patentansprüche

1. Verfahren zur Herstellung von Lauryllactam, bei dem man eine Beckmann-Umlagerung von Cyclododecanonoxim in der Gasphase bei einer Temperatur zwischen 180 und 450°C und einem Druck zwischen 50 und 700 mbar absolut in Gegenwart eines mikroporösen Materials mit einer dreidimensionalen anorganischen Hauptstruktur aus über eine gemeinsame Ecke verbundenen Tetraedern, das als Zeolith bezeichnet wird, durchführt.

2. Verfahren nach Anspruch 1, bei dem es sich bei dem Zeolith um einen Zeolith mit einer Porenverteilung im Bereich großer Poren handelt.

3. Verfahren nach Anspruch 1 oder 2, bei dem:
a) das Cyclododecanonoxim entweder in einem aus Alkoholen und Kohlenwasserstoffen ausgewählten Lösungsmittel gelöst wird oder in geschmolzenen Zustand vorliegt,
b) der Strom aus Schritt a) verdampft und gegebenenfalls mit Hilfe eines Trägergases mit dem Zeolith in Berührung gebracht wird,
c) das Lauryllactam von dem Lösungsmittel, dem gegebenenfalls vorhandenen Trägergas und dem gegebenenfalls vorhandenen nicht umgesetzten Cyclododecanonoxim getrennt wird.

4. Verfahren nach einem der Ansprüche 1 bis 3, bei dem die Temperatur der Umlagerung zwischen 225 und 400°C liegt.

5. Verfahren nach Anspruch 4, bei dem die Temperatur zwischen 225 und 375°C liegt.

6. Verfahren nach einem der vorhergehenden Ansprüche, bei dem es sich bei dem Zeolith um einen USY-Zeolith handelt.

7. Verfahren nach einem der Ansprüche 1 bis 5, bei dem man den Zeolith aus einem Zeolith, der Aluminium und/oder Bor als Gerüstheteroatom aufweist, einem Zeolith, der anfänglich Aluminium und/oder Bor als Gerüstheteroatom aufweist und einer Desaluminierungs-/Deborierungsbehandlung unterworfen worden ist, einem β-Zeolith, einem β-Zeolith mit Aluminium und/oder Bor als Gerüstheteroatom, einem β-Zeolith, der anfänglich Aluminium und/oder Bor als Gerüstheteroatom aufweist und einer Desaluminierungs-/Deborierungsbehandlung unterworfen worden ist, auswählt.

8. Verfahren nach Anspruch 7, bei dem es sich bei dem Zeolith um einen Zeolith, der anfänglich Aluminium und/oder Bor als Gerüstheteroatom aufweist und einer Desaluminierungs-/Deborierungsbehandlung unterworfen worden ist, handelt.

9. Verfahren nach Anspruch 7, bei dem es sich bei dem Zeolith um einen β-Zeolith, der anfänglich Aluminium und/oder Bor als Gerüstheteroatom aufweist und einer Desaluminierungs-/Deborierungs-behandlung unterworfen worden ist, handelt.

10. Verfahren nach Anspruch 8 oder 9, bei dem der Zeolith, gegebenenfalls der Beta-Zeolith, der anfänglich Aluminium als Gerüstheteroatom aufweist, einer Desaluminierung unterworfen wird, die zu einem Si/Al-Atomverhältnis von mehr als 50 führt.

11. Verfahren nach Anspruch 10, bei dem der Zeolith, gegebenenfalls der Beta-Zeolith, der anfänglich das Aluminiumatom als Gerüstheteroatom aufweist, einer Desaluminierung unterworfen wird, die zu einem Si/Al-Atomverhältnis von mehr als 80 führt.

12. Verfahren nach Anspruch 11, bei dem der Zeolith, gegebenenfalls der Beta-Zeolith, der anfänglich das Aluminiumatom als Gerüstheteroatom aufweist, einer Desaluminierung unterworfen wird, die zu einem Si/Al-Atomverhältnis von mehr als 150 führt.

13. Verfahren nach Anspruch 8 oder 9, bei dem der Zeolith, gegebenenfalls der Beta-Zeolith, der anfänglich das Boratom als Gerüstheteroatom aufweist, einer Deborierung unterworfen wird, die zu einem Si/B-Atomverhältnis von mehr als 20 führt.

14. Verfahren nach Anspruch 13, bei dem der Zeolith, gegebenenfalls der Beta-Zeolith, der anfänglich das Boratom als Gerüstheteroatom aufweist, einer Deborierung unterworfen wird, die zu einem Si/B-Atomverhältnis von mehr als 40 führt.

15. Verfahren nach Anspruch 8 oder 9, bei dem der Zeolith, gegebenenfalls der Beta-Zeolith, der anfänglich das Aluminiumatom und das Boratom als Gerüstheteroatom aufweist, einer Desaluminierung/Deborierung unterworfen wird, die zu einem Si/Al-Verhältnis von mehr als 50 und einem Si/B-Verhältnis von mehr als 20 führt.

16. Verfahren nach Anspruch 15, bei dem der Zeolith, gegebenenfalls der Beta-Zeolith, der anfänglich das Aluminiumatom und das Boratom als Gerüstheteroatom aufweist, einer Desaluminierung/Deborierung unterworfen wird, die zu einem Si/Al-Verhältnis von mehr als 150 und einem Si/B-Verhältnis von mehr als 30 führt.

17. Verfahren nach einem der Ansprüche 3 bis 16, bei dem das Cyclododecanonoxim in geschmolzenem Zustand eingetragen wird.

18. Verfahren nach einem der Ansprüche 3 bis 16, bei dem das Cyclododecanonoxim in einem aus Alkoholen und Kohlenwasserstoffen ausgewählten Lösungsmittel gelöst wird und der Alkohol aus Methanol, Ethanol und Isopropanol ausgewählt wird.

19. Verfahren nach Anspruch 18, bei dem das Lösungsmittel aus Isopropanol/Cyclohexan- und Ethanol/Cyclohexan-Mischungen ausgewählt wird.

20. Verfahren nach einem der vorhergehenden Ansprüche, bei dem eine Regeneration des Zeoliths durch-geführt wird.

21. Verfahren nach Anspruch 20, bei dem eine Regeneration des Zeoliths durchgeführt wird, sobald eine Abnahme der Ausbeute zwischen 10 und 20% beobachtet wird.

## Claims

1. Process for the preparation of lauryllactam in which a Beckmann rearrangement of cyclododecanone oxime is carried out in the gas phase at a temperature of between 180 and 450°C, at a pressure of between 50 and 700 mbar absolute, in the presence of a microporous material having a three-dimensional inorganic main structure composed of tetrahedra connected via a common edge, called zeolite.

2. Process according to Claim 1, in which the zeolite is a zeolite exhibiting a pore distribution within the region of the wide pores.

3. Process according to Claim 1 or 2, in which:
a) the cyclododecanone oxime is either dissolved in a solvent chosen from alcohols and hydrocarbons or is in the molten state,
b) the stream from stage a) is vaporized and brought into contact with the zeolite, optionally using a carrier gas,
c) the lauryllactam is separated from the solvent, from the possible carrier gas and from the possible cyclododecanone oxime which has not been converted.

4. Process according to any one of Claims 1 to 3, in which the temperature of the rearrangement is between 225 and 400°C.

5. Process according to Claim 4, in which the temperature is between 225 and 375°C.

6. Process according to any one of the preceding claims, in which the zeolite is a USY zeolite.

7. Process according to any one of Claims 1 to 5, in which the zeolite is chosen from a zeolite which has aluminium and/or boron as framework heteroatom, a zeolite which initially has aluminium and/or boron as framework heteroatom and which has been subjected to a dealumination/deboration treatment, a β zeolite, a β zeolite which has aluminium and/or boron as framework heteroatom or a β zeolite which initially has aluminium and/or boron as framework heteroatom and which has been subjected to a dealumination/deboration treatment.

8. Process according to Claim 7, in which the zeolite is a zeolite which initially has aluminium and/or boron as framework heteroatom and which has been subjected to a dealumination/deboration treatment.

9. Process according to Claim 7, in which the zeolite is a β zeolite which initially has aluminium and/or boron as framework heteroatom and which has been subjected to a dealumination/deboration treatment.

10. Process according to Claim 8 or 9, in which the optionally β zeolite which initially has aluminium as framework heteroatom is subjected to a dealumination resulting in an Si/Al atomic ratio of greater than 50.

11. Process according to Claim 10, in which the option-ally β zeolite which initially has the aluminium atom as framework heteroatom is subjected to a dealumination resulting in an Si/Al atomic ratio of greater than 80.

12. Process according to Claim 11, in which the optionally β zeolite which initially has the aluminium atom as framework heteroatom is subjected to a dealumination resulting in an Si/Al atomic ratio of greater than 150.

13. Process according to Claim 8 or 9, in which the optionally β zeolite which initially has the boron atom as framework heteroatom is subjected to a deboration resulting in an Si/B atomic ratio of greater than 20.

14. Process according to Claim 13, in which the optionally β zeolite which initially has the boron atom as framework heteroatom is subjected to a deboration resulting in an Si/B atomic ratio of greater than 40.

15. Process according to Claim 8 or 9, in which the optionally β zeolite which initially has the aluminium atom and the boron atom as framework heteroatom is subjected to a dealumination/deboration resulting in an Si/Al ratio of greater than 50 and an Si/B ratio of greater than 20.

16. Process according to Claim 15, in which the optionally β zeolite which initially has the aluminium atom and the boron atom as framework heteroatom is subjected to a dealumination/deboration resulting in an Si/Al ratio of greater than 150 and an Si/B ratio of greater than 30.

17. Process according to any one of Claims 3 to 16, in which the cyclododecanone oxime is introduced in the molten state.

18. Process according to any one of Claims 3 to 16, in which the cyclododecanone oxime is dissolved in a solvent chosen from alcohols and hydrocarbons and the alcohol is chosen from methanol, ethanol or isopropanol.

19. Process according to Claim 18, in which the solvent is chosen from isopropanol/cyclohexane and ethanol/cyclohexane mixtures.

20. Process according to any one of the preceding claims, in which the zeolite is regenerated.

21. Process according to Claim 20, in which the zeolite is regenerated as soon as a fall in the yield of between 10 and 20% is observed.
